# EUROPÄISCHE PATENTANMELDUNG

(11) **EP 1 240 910 A2**
(43) Veröffentlichungstag der Anmeldung: **18.09.2002**
(21) Anmeldenummer: 01128506.1
(22) Anmeldetag: 29.11.2001
(51) Int. Cl.: A61M 1/16

(54) **Salzbehältersystem für Dialysegeräte**

(30) Priorität: 08.01.2001 DE 10100549
(71) Anmelder: Ritter, Franz Peter, 86636 Untermeitingen (DE)
(72) Erfinder: Ritter, Franz Peter, 86636 Untermeitingen (DE)
(74) Vertreter: Gallo, Wolfgang, Dipl.-Ing. (FH)

(57) **Zusammenfassung**

Salzbehältersystem für Dialysegeräte, bestehend aus einem flexiblen Beutel (1) als Salzbehälter mit zwei an gegenüberliegenden Enden angeordneten Anschlußelementen (12 bis 19) und einem steifen Stützbügel (2), der mit dem Beutel kuppelbar ist und dessen beidendige Anschlußelemente in definiertem Abstand hält.

## Beschreibung

Bei Dialysegeräten für die Blutwäsche wird das zur Zubereitung der Dialysierflüssigkeit im Dialysegerät notwendige Salz in Form von Trockenkonzentratpulver in jeweils einem Dialysevorgang entsprechender Menge in Behältern bereitgestellt und verwendet, die mit entsprechenden Anschlüssen des Dialysegeräts kuppelbare Anschlüsse haben, um den Trockenkonzentratbehälter zur Verwendung des Trockenkonzentratpulvers mit dem Flüssigkeitskreislauf des Dialysegeräts zu verbinden.

Eine gebräuchliche Form solcher Trockenkonzentratbehälter sind Beutel, an denen ein integrierter Stecker angeordnet ist, der mit einem Gegenstecker des Dialysegeräts verbindbar ist und einen in das Beutelinnere führenden Flüssigkeitseinlaß und einen aus dem Beutel herausführenden Flüssigkeitsauslaß aufweist.

Eine weitere gebräuchliche Form solcher Trockenkonzentratbeutel sind etwa zylindrische steife Kartuschen, die am Boden und am Deckel axial miteinander fluchtend angeordnete Anschlußelemente haben, die mit entsprechenden Gegenelementen des Dialysegeräts zusammenwirken und von denen einer als Flüssigkeitseinlaß und der andere als Flüssigkeitsauslaß aus dem Behälter dient. Dabei haben die Anschlußelemente der Trockenkonzentratkartusche typischerweise durchstechbare Membranen, die beim Verbinden mit den Gegenelementen des Dialysegeräts durchstochen werden, um so die Strömungsverbindung herzustellen.

Die Trockenkonzentratbehälter in Form flexibler Beutel haben den Vorteil, daß sie im entleerten Zustand nach Gebrauch zusammendrückbar sind und ein geringes Abfallvolumen haben. Sie haben aber den Nachteil, daß sie aufwendig in der Herstellung sind, weil der Beutel selbst aus flexiblem Kunststoff besteht, der mit einem steifen Steckerteil ebenfalls aus Kunststoff verbunden ist und außerdem vom Steckerteil noch ein Schlauch in das Beutelinnere in den vom Steckerteil entfernten Beutelendbereich eingesetzt werden muß, um eine Flüssigkeitsrezirkulation durch den gesamten Beutel zu erreichen.

Die Trockenkonzentratbehälter in Form steifer Kartuschen haben zwar den Vorteil, daß sie aus einheitlichem Kunststoff im Spritzgußverfahren günstig herstellbar sind und, weil ihre Anschlußelemente an Boden und Deckel einander gegenüberliegend angeordnet sind, der Flüssigkeitskreislauf im Gebrauch durch den Behälter problemlos und ohne besondere Maßnahmen erfolgen kann, aber die steifen Behälter haben den Nachteil, daß sie nach Gebrauch im entleerten Zustand sperrig und voluminös sind und deshalb ein großes Abfallvolumen verkörpern.

Der Erfindung liegt die Aufgabe zugrunde, ein Salzbehältersystem zur Aufnahme des Trockenkonzentratpulvers zur Verwendung in Dialysegeräten zu schaffen, welches die Vorteile der flexiblen Beutel im Hinblick auf ein geringes Abfallvolumen nach Gebrauch mit den Vorteilen der steifen Kartuschen mit axial fluchtend an entgegengesetzten Enden liegenden Anschlußelementen in sich vereinigt.

Diese Aufgabe wird gemäß der Erfindung durch das im Anspruch 1 angegebene Salzbehältersystem gelöst. Vorteilhafte Ausgestaltungen der Erfindung sind Gegenstand der Unteransprüche.

Das erfindungsgemäße Behältersystem besteht also aus dem eigentlichen Trockenkonzentratbehälter in Gestalt eines Beutels mit an entgegengesetzten Enden angeordneten Anschlußelementen und einem gesonderten steifen Stützbügel, mit welchem der Beutel zum Gebrauch verbindbar ist und der dann die beiden gegenüberliegenden Anschlußelemente des Beutels in definiertem Abstand voneinander hält, so daß die Beutel-Stützbügel-Kombination wie eine steife Kartusche in das Dialysegerät einsetzbar ist, ohne daß beim Schließen der Anschlußmechanik des Dialysegeräts das obere Anschlußelement des flexiblem Beutels nach unten weggedrückt werden kann.

Der Stützbügel kann dabei mit einem jeweils neuen Trockenkonzentratbeutel immer wieder verwendet werden.

Nach Gebrauch verbleibt als Abfall also nur der leere Beutel, der flexibel und flach zusammendrückbar ist und dessen beide Anschlußelemente im Gegensatz zu dem voluminösen Steckerteil herkömmlicher Trockenkonzentratbeutel sehr kompakt sind.

Der Beutel selbst kann jede beliebige Form haben, also beispielsweise rechteckig, aber auch tetraederförmig. Die letztere Form hat den Vorteil, ein sehr kompaktes Stapeln gefüllter Beutel zu ermöglichen.

Die Erfindung wird im folgenden anhand eines Ausführungsbeispiels unter Bezugnahme auf die anliegenden Zeichnungen mehr im einzelnen beschrieben, in welchen zeigt:
- Fig. 1: ein Salzbehältersystem nach der Erfindung in der Aufnahmevorrichtung eines Dialysegeräts (wobei das Dialysegerät selbst nicht dargestellt ist),
- Fig. 2: den Trockenkonzentratbeutel des Salzbehältersystems in Seitenansicht,
- Fig. 3: den Trockenkonzentratbeutel nach Fig. 2 in Stirnansicht,
- Fig. 4: eine erste Ausführungsform des Stützbügels in Seitenansicht,
- Fig. 5: den Stützbügel nach Fig. 4 in Draufsicht, und
- Fig. 6: eine alternative Ausführungsform des Stützbügels mit eingesetztem Trockenkonzentratbeutel in geschnittener Frontansicht.

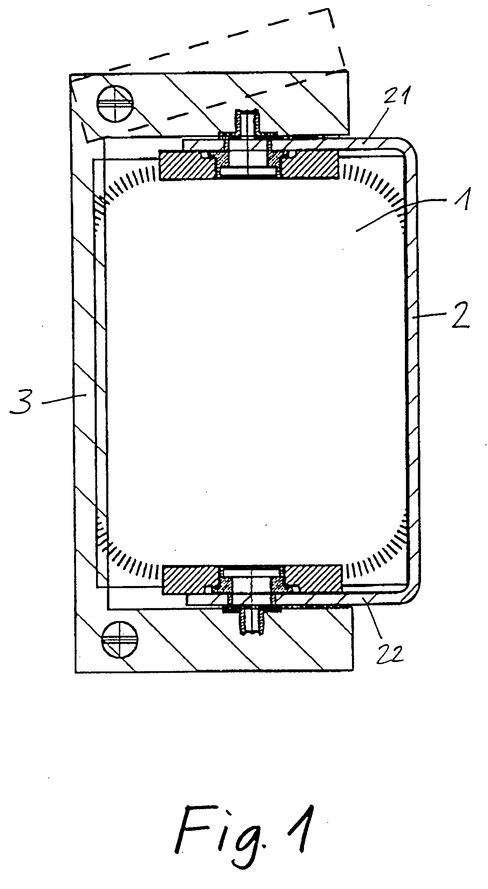

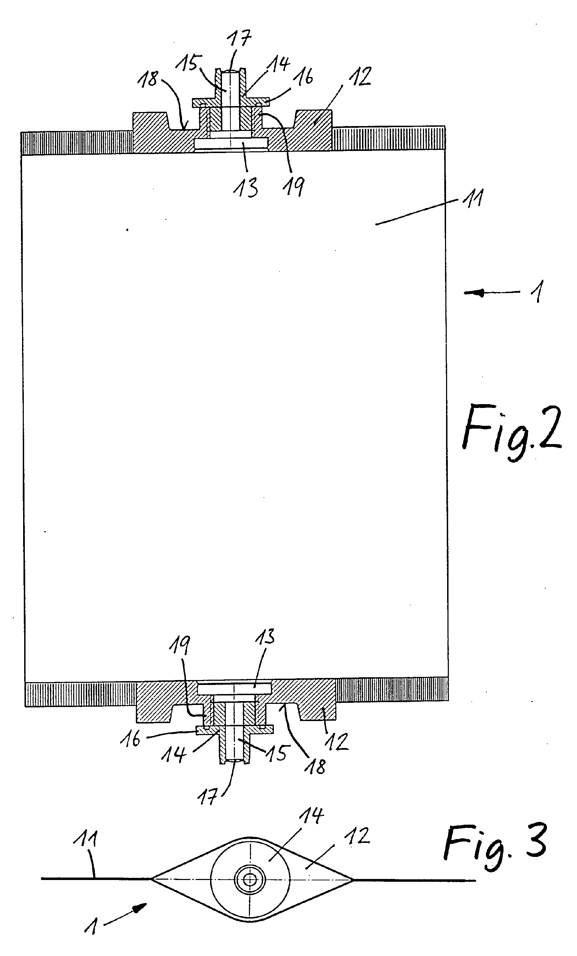

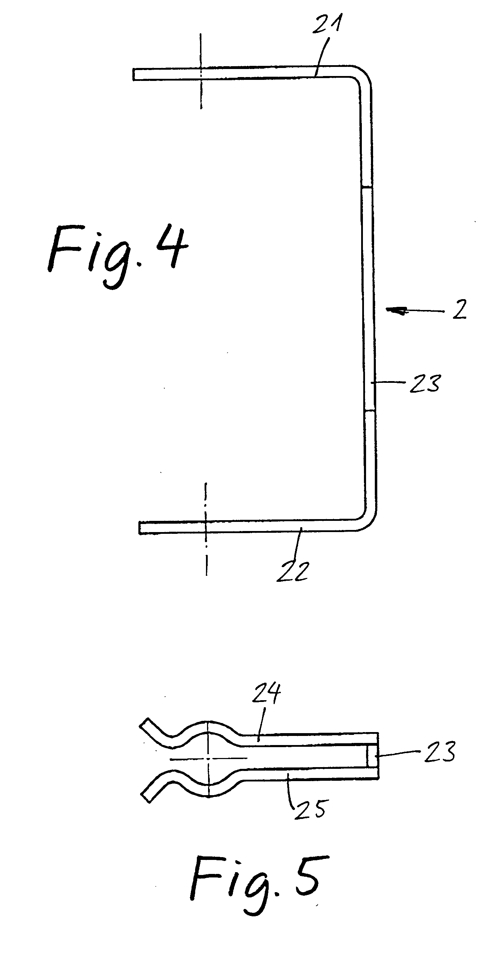

Fig. 1 zeigt das erfindungsgemäße Salzbehältersystem, bestehend aus einem Beutel 1, wie er in den Fig. 2 und 3 im einzelnen dargestellt ist, und einem Stützbügel 2 der in den Fig. 4 und 5 dargestellten Ausführungsform in der Aufnahmevorrichtung 3 eines im übrigen nicht dargestellten Dialysegeräts.

Die Fig. 2 und 3 zeigen den Beutel 1 in Frontansicht mit axial geschnittenen Anschlußelementen am oberen und unteren Beutelende bzw. in Stirnansicht, wobei der Beutel jeweils im ungefüllten (oder entleerten) Zustand mit flach zusammengelegtem Beutelkörper dargestellt ist.

Daraus ist ersichtlich, daß der Beutelkörper 11 bei dieser Beutelausführungsform rechteckförmig ist. Der Beutelkörper kann aber selbstverständlich auch jede beliebige andere Form haben, beispielsweise schlauchförmig oder, wie oben schon erwähnt, tetraederförmig.

An beiden Beutelenden, also oben und unten, ist jeweils mittig ein Anschlußelement gebildet, das jeweils aus einem mit dem Beutelkörper 11 verbundenen Anschlußkörper 12 mit einer mittigen Bohrung 13 und einem darin eingesetzten, nämlich beispielsweise eingeschraubten oder eingeschweißten Anschlußnippel 14 mit mittiger Bohrung 15, einem scheibenförmigen Radialflansch 16 und einer durchstechbaren Verschlußmembran 17 besteht. Wie man aus der Zeichnung sieht, hat der jeweilige Anschlußkörper 12 eine mittige querverlaufende äußere Vertiefung 18, in deren Mitte ein die Bohrung 13 umgebender zylindrischer Halsansatz 19 nach außen vorspringt, auf welchem der scheibenförmige Radialflansch 16 des Anschlußnippels 14 aufsitzt. Dabei steht der Radialflansch 16 des Anschlußnippels 14 radial über den Halsansatz 19 des Anschlußkörpers 12 über.

Die Fig. 4 und 5 zeigen den zum Gebrauch des gefüllten Trockenkonzentratbeutels 1 zu verwendenden und mit dem Beutel zu kuppelnden Stützbügel 2 in Seitenansicht bzw. Draufsicht. In der Seitenansicht hat der Stützbügel zwei eine U-Form mit einem oberen und einem unteren horizontalen Schenkel 21 bzw. 22 und einem dazwischen vertikal verlaufenden Mittelteil 23. Der Mittelteil 23 dient zur starren und abstandswahrenden Verbindung der beiden horizontalen Schenkel 21 und 22, welch letztere gleich ausgebildet sind (so daß der Stützbügel keiner bestimmten Orientierung bedarf), und diese Schenkel sind, wie aus Fig. 5 hervorgeht, jeweils als clipartige Klammern mit zwei spiegelbildlich ausgebildeten Klammerschenkeln 24 und 25 ausgebildet.

Der Stützbügel 2 wird zum Gebrauch in der aus Fig. 1 ersichtlichen Weise mit dem Trockenkonzentratbeutel 1 zusammengesteckt, wobei die beiden Klammerschenkel 24, 25 des oberen und unteren Schenkels jeweils den Halsansatz 19 jedes Anschlußelements umgreifen und dieses rastend umfaßt und dabei außerdem den radial überstehenden scheibenförmigen Radialflansch 16 des Anschlußnippels 14 untergreifen. Der starre Stützbügel 1 hält also den damit kombinierten Beutel 1 in gestreckter Position, so daß die beiden Anschlußelemente des Beutels an dessen oberem bzw. unterem Ende (beide sind identisch) in definiertem Abstand gehalten werden, so daß beim Schließen des Aufnahmemechanismus 3 des Dialysegeräts durch herunterschwenken von dessen beweglichem oberen Teil (in Fig. 1 in der geöffneten Stellung gestrichelt angedeutet), wobei die Verschlußmembranen 17 der beiden Anschlußelemente von den Gegenelementen der Aufnahmevorrichtung durchstochen werden, nicht zusammengedrückt werden können, also nicht unter der zum Durchstechen und dichten Zusammenstecken angewendeten Axialkraft ausweichen können.

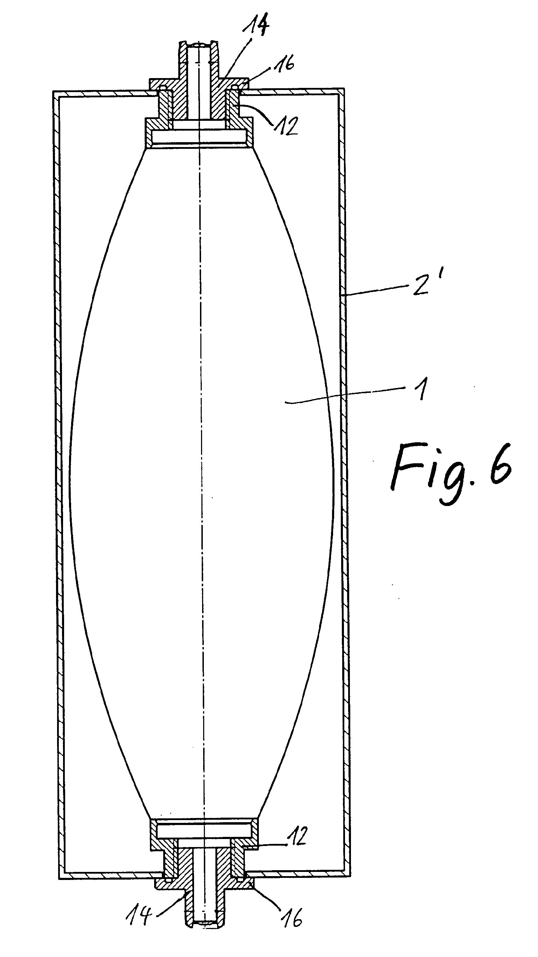

Fig. 6 zeigt eine Variante des Stützbügels mit eingesetztem Trockenkonzentratbeutel 1, wobei der Stützbügel 2' eine Rohroder Kastenkonfiguration hat, die oben und unten bis auf einen zur Aufnahme des jeweiligen Anschlußelements des Beutels dienenden Schlitz geschlossen ist und an einer Seite offen ist, um den Beutel einsetzen zu können, wobei die Schlitzränder jeweils wieder einen Radialflansch 16 des oberen bzw. unteren Anschlußnippels 14 des jeweiligen Anschlußkörpers 12 des Beutels 1 abstandswahrend untergreifen.

## Patentansprüche

1. Salzkonzentratbehältersystem für Dialysegeräte, bestehend aus einem flexiblen Beutel (1) als Salzbehälter, der an zwei gegenüberliegenden Enden mit jeweils einem Anschlußelement (12 bis 19) zur Verbindung mit einem Gegenelement einer Aufnahmevorrichtung eines Dialysegeräts versehen ist, und mit einem steifen Stützbügel (2), der mit den beiden Anschlußelementen des Beutels (1) kuppelbar ist und diese in definiertem Abstand hält.

2. Salzbehältersystem nach Anspruch 1, wobei der Stützbügel (2) zwei durch ein Verbindungsstück (23) verbundene klammerartige Schenkel (21, 22) aufweist, die mit den beiden Anschlußelementen (14, 16) verrastbar sind.

3. Salzbehältersystem nach Anspruch 1, wobei der Stützbügel als an einer Seite offene Rohr- oder Kastenkonstruktion mit an beiden axialen Enden gebildeten Stirnwänden mit Schlitzöffnungen bzw. Elementen zur Aufnahme jeweils eines Anschlußelements des Beutels (1) ausgebildet ist.

4. Salzbehältersystem nach einem der Ansprüche 1 bis 3, wobei der Stützbügel (2) jeweils einen Ringbund bzw. Ringflansch (16) des jeweiligen Anschlußelements des Beutels untergreift.
